# EUROPEAN PATENT APPLICATION

(11) **EP 4 582 788 A1**
(43) Date of publication of application: **09.07.2025**
(21) Application number: 22963608.9
(22) Date of filing: 14.11.2022
(51) Int. Cl.: G01N 15/06, G01N 21/49, G01N 33/18

(54) **DEVICE AND METHOD FOR MEASURING TURBIDITY**

(30) Priority: 24.10.2022 KR 20220137662
(71) Applicant: LG Electronics Inc., Yeongdeungpo-gu Seoul 07336 (KR)
(72) Inventor: CHO, Jeonghoon, Seoul 06772 (KR); LEE, Kihyuk, Seoul 06772 (KR)
(74) Representative: Vossius & Partner Patentanwälte Rechtsanwälte mbB
(86) International application number: PCT/KR2022/017871
(87) International publication number: WO 2024/090647

(57) **Abstract**

The present invention relates to a device and a method capable of measuring turbidity of water used in home appliances, the device comprising: a fluid storage part comprising a reflector; a first light source that emits light to the fluid inside the fluid storage part; a first light receiving part that receives scattered light scattered by suspended particles in the fluid; and a control part that measures the turbidity of the fluid by controlling the first light source and the first light receiving part, wherein the first light source and the first light receiving part are positioned to be spaced apart from each other at a predetermined angle around the fluid storage part in the vicinity of the fluid storage part, and wherein, among surfaces of the fluid storage part, the reflector may be positioned between a first surface facing the first light source and a second surface facing the first light receiving part.

## Description

### TECHNICAL FIELD

The present disclosure relates to a device and method for measuring turbidity of water used in home appliances.

### BACKGROUND ART

In general, home appliances that use water, such as water purifiers, dishwashers, and washing machines, have to use clean water, and thus, various sensors for monitoring turbidity of water have to be mounted.

Turbidity refers to a concentration of light-scattering particles or light-absorbing particles suspended in a fluid. When the turbidity increases in the fluid, light transmittance can vary depending on a distribution of suspended particles in the fluid, a refractive index, surface properties, etc.

Such turbidity information of water can be used to minimize waste of water, electricity, detergent, etc. by changing a washing cycle or purification cycle of home appliances, and to provide drinking water purified under optimal conditions, or to provide items such as tableware and clothing washed under optimal conditions.

However, existing turbidity sensors have limitations in measuring water quality pollution in low turbidity regions, making it difficult to ensure safety of drinking water, such as in drinking water appliances.

Therefore, in the future, it is necessary to develop a device for measuring turbidity with a broadband sensing function that can detect water quality in not only high turbidity regions but also low turbidity regions.

### DISCLOSURE OF THE INVENTION

### TECHNICAL PROBLEM

An object of the present disclosure is to solve the above-mentioned problems and other problems.

An object of the present disclosure is to provide a device and method for measuring turbidity, which are capable of enabling broadband sensing for measuring water quality from a low turbidity region to a high turbidity region by amplifying a low turbidity optical signal using a reflector.

### TECHNICAL SOLUTION

A device for measuring turbidity according to an embodiment of the present disclosure can include: a fluid storage part including a reflector; a first light source configured to emit light to a fluid within the fluid storage part; a first light receiving part configured to receive scattered light scattered by suspended particles in the fluid; and a control part configured to measure turbidity of the fluid by controlling the first light source and the first light receiving part, wherein the first light source and the first light receiving part are disposed to be spaced a predetermined angle from each other around the fluid storage part in the vicinity of the fluid storage part, and the reflector is disposed between a first surface facing the first light source and a second surface facing the first light receiving part on a surface of the fluid storage part.

In an embodiment, the device can further include a second light source configured to emit light to the fluid within the fluid storage part, wherein the second light source can be spaced a predetermined angle from the first light source around the fluid storage part and disposed to face the first light receiving part.

In an embodiment, the control part can be configured to: receive a first light receiving signal of the first light receiving part when the first light source is turned on, and the second light source is turned off; receive a second light receiving signal of the first light receiving part when the first light source is turned off, and the second light source is turned on; and measure the turbidity of the fluid on the basis of the first light receiving signal and the second light receiving signal.

In an embodiment, when the turbidity of the fluid is measured, the control part can be configured to classify the turbidity of the fluid into a high turbidity region to measure a high turbidity value of the fluid on the basis of the second light receiving signal when there is no change in the first light receiving signal due to a saturation state of the first light receiving part, and the second light receiving signal is normally received.

In an embodiment, when the turbidity of the fluid is measured, the control part can be configured to classify the turbidity of the fluid into a low turbidity region to measure a low turbidity value of the fluid on the basis of the first light receiving signal when there is no change in the second light receiving signal due to a saturation state of the first light receiving part, and the second light receiving signal is normally received.

In an embodiment, the device can further include a second light receiving part configured to receive the scattered light scattered by the suspended particles of the fluid, wherein the second light receiving part can be spaced a predetermined angle from the first light receiving part around the fluid storage part and disposed to face the first light receiving part.

A method for measuring turbidity using a device for measuring turbidity according to an embodiment of the present disclosure can include: receiving an user input requesting a turbidity measurement; turning on the first light source and turning off the second light source when the user input is received; receiving a first light receiving signal from the light receiving part: turning off the first light source and turning on the second light source; receiving a second light receiving signal from the light receiving part: and measuring the turbidity of the fluid on the basis of the first light receiving signal and the second light receiving signal.

### ADVANTAGEOUS EFFECTS

According to the embodiment of the present disclosure, the device for measuring the turbidity can amplify the optical signal of the low turbidity using the reflector to enable the broadband sensing for measuring the water quality from the low turbidity region to the high turbidity region.

In addition, in the present disclosure, the safety of the drinking water can be secured by sensing the low turbidity of the water quality in the water-containing home appliances using water.

In addition, in the present disclosure, since the customized filter replacement is enabled according to the water quality pollution level, the unnecessary replacement costs can be reduced by setting the customized filter replacement cycle according to the pollution standard.

In addition, the present disclosure can be applied to the home appliances such as the water purifiers and the dishwashers that utilize the various water qualities with the broadband sensing function.

In addition, in the present disclosure, since the light source is implemented using the low-price LED, the low-price sensor can be provided and be expanded to the various home appliances.

In addition, the present disclosure can provide the water quality control and the customer assurance service through the real-time water quality monitoring and measurement.

### BRIEF DESCRIPTION OF THE DRAWINGS

FIG. 1 is a view for explaining a device for measuring turbidity according to an embodiment of the present disclosure.
FIG. 2 is a cross-sectional view taken along line I-I' of FIG. 1.
FIGS. 3 to 5 are views for explaining a fluid storage part of the device for measuring the turbidity according to an embodiment of the present disclosure.
FIG. 6 is a view for explaining a reflector of the device for measuring the turbidity according to an embodiment of the present disclosure.
FIG. 7 is a view for explaining a method for measuring turbidity in the device for measuring the turbidity according to an embodiment of the present disclosure.
FIG. 8 is a view for explaining a device for measuring turbidity according to another embodiment of the present disclosure.
FIG. 9 is a view for explaining a method for measuring turbidity in the device for measuring the turbidity according to another embodiment of the present disclosure.
FIG. 10 is a view for explaining position setting of the reflector of the device for measuring the turbidity according to an embodiment of the present disclosure.
FIG. 11 is a view for explaining position setting of a light receiving part of the device for measuring the turbidity according to an embodiment of the present disclosure.
FIGS. 12 and 13 are views for explaining corresponding optical signal amplification before and after the reflector is applied to the device for measuring the turbidity according to an embodiment of the present disclosure.
FIG. 14 is a view for explaining a method for measuring turbidity in the device for measuring the turbidity according to an embodiment of the present disclosure.

### MODE FOR CARRYING OUT THE INVENTION

Hereinafter, embodiments disclosed in this specification is described with reference to the accompanying drawings, and the same or corresponding components are given with the same drawing number regardless of reference number, and their duplicated description will be omitted. The suffixes "module" and "part" for components used in the description below are assigned or mixed in consideration of easiness in writing the specification and do not have distinctive meanings or roles by themselves. Moreover, detailed descriptions related to well-known functions or configurations will be ruled out in order not to unnecessarily obscure subject matters of the present disclosure. However, this does not limit the present disclosure within specific embodiments and it should be understood that the present disclosure covers all the modifications, equivalents, and replacements within the idea and technical scope of the present disclosure.

It will be understood that although the ordinal numbers such as first and second are used herein to describe various elements, these elements should not be limited by these numbers. The terms are only used to distinguish one component from other components.

It will also be understood that when an element is referred to as being "'connected to" or "engaged with" another element, it can be directly connected to the other element, or intervening elements can also be present. It will also be understood that when an element is referred to as being 'directly connected to' another element, there is no intervening elements.

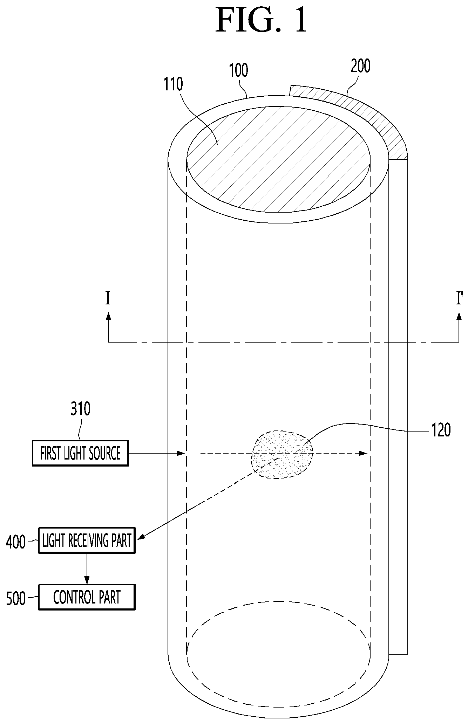

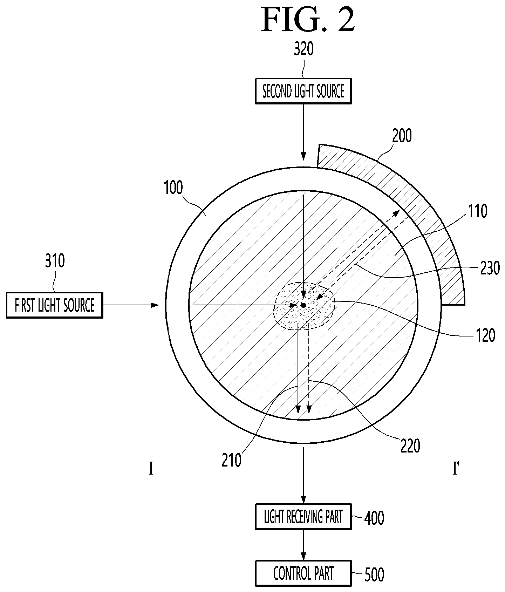

FIG. 1 is a view for explaining a device for measuring turbidity according to an embodiment of the present disclosure, and FIG. 2 is a cross-sectional view taken along line I-I' of FIG. 1.

As illustrated in FIGS. 1 and 2, a device for measuring turbidity of the present disclosure can include a fluid storage part 100 including a reflector 200, a first light source 310 and a second light source 320, which emit light into a fluid 110 inside the fluid storage part 100, a light receiving part 400 that receives scattered light scattered by suspended particles 120 of the fluid 110, and a control part 500 that controls the first light source 310, the second light source 320, and the light receiving part 400 to measure turbidity of the fluid 110.

Here, the fluid storage part 100 can have a cylindrical shape in which the fluid is stored.

In some cases, the fluid storage part 100 can have a pipe shape having a through-hole defined therein so that the fluid can flow, but this is only an example and is not limited thereto.

In addition, an entire surface of the fluid storage part 100 can be provided as a light-transmitting member.

The reason is to allow light emitted from an external light source to be incident into the internal fluid of the fluid storage part 100 and to receive internal scattered light.

In some cases, only some surfaces of the fluid storage part 100 can be provided as the light-transmitting member.

For example, the fluid storage part 100 can have the light-transmitting member only on an incident surface, through which light is incident inward from the outside, and an emission surface, through which the internal scattered light is emitted to the outside.

Here, the reflector 200 can be attached to an inner surface of the fluid storage part 100.

As another example, the fluid storage part 100 can have the light-transmitting member only on an incident surface, through which light is incident inward from the outside, an emission surface, through which the internal scattered light is emitted to the outside, and an attachment surface to which the reflector 200 is attached.

Here, the reflector 200 can be attached to an outer surface of the fluid storage part 100.

The reflector 200 can amplify the scattered light by reflecting or re-reflecting the scattered light scattered by the suspended particles inside the fluid storage part 100 toward the suspended particles inside the fluid storage part 100.

That is, the reflector 200 can maximize light reflection characteristics of low turbidity particles with low light scattering.

Here, the reflector 200 can be disposed between a first surface of the fluid storage part 100 facing the first light source 310 and a second surface facing the light receiving part 400.

The reason is that, when the reflector 200 is disposed on an area between the first surface facing the first light source 310 and the second surface facing the light receiving part 400 on a surface of the fluid storage part 100, a light receiving signal of the light receiving part 400, by which a turbidity variation and low turbidity distinction are maximized, can be obtained.

Thus, in the present disclosure, water quality in a low turbidity region can be accurately measured by analyzing the light receiving signal of the light receiving part 400, by which the turbidity variation and the low turbidity distinction are maximized.

For example, the reflector 200 can be at least one of a retro-reflective film or a light reflective film, but this is only an example and is not limited thereto.

In addition, the reflector 200 can amplify the scattered light by reflecting or re-reflecting the scattered light onto the suspended particles 120 of the fluid 110.

Here, the reflector 200 can amplify the scattered light by reflecting or re-reflecting the scattered light toward the suspended particles 120 disposed at an inner center of the fluid storage part 100.

For example, a length of the reflector 200 can be less than or equal to a length of the fluid storage part 100, and a width of the reflector 200 can be less than or equal to a width between the first surface of the fluid storage part 100 facing the first light source 310 and the second surface of the fluid storage part 100 facing the light receiving part 400.

Here, an area of the reflector 200 can be calculated by a formula expressed by: S = L × W (S is an area of the reflector, L is a length of the fluid storage part, and W is a width between the first surface and the second surface of the fluid storage part), but this is only an example and is not limited thereto.

Next, the first light source 310 and the light receiving part 400 can be disposed to be spaced a predetermined angle from each other around the fluid storage part 100 by using the fluid storage part 100 as a center.

For example, the first light source 310 and the light receiving part 400 can be disposed in a direction perpendicular to each other by using the fluid storage part 100 as the center.

In addition, the second light source 320 can be disposed to be spaced a predetermined angle from the first light source 310 around the fluid storage part 100 and face the light receiving part 400.

For example, the second light source 320 can be perpendicular to the first light source 310 with respect to the fluid storage part 100.

In addition, the second light source 320 and the light receiving part 400 can be disposed symmetrically at both sides of the fluid storage part 100 along a line passing through a center point of the fluid storage part 100.

In addition, the second light source 320 can be disposed on a second line having a predetermined angle with respect to the first line while passing through the center point of the fluid storage part 100 when the first light source 310 is disposed on the first line passing through the center point of the fluid storage part 100.

For example, the second light source 320 can be disposed on a second line perpendicular to the first line while passing through the center point of the fluid storage part 100.

In addition, a light output intensity of the second light source 320 can be the same as a light output intensity of the first light source 310.

In some cases, the light output intensity of the second light source 320 can be different from the light output intensity of the first light source 310.

Next, each of the first light source 310 and the second light source 320 can include a light-emitting diode, but this is only one embodiment and is not limited thereto.

Next, the control part 500 can alternately switch the first light source 310 and the second light source 320 so that, when the first light source 310 is turned on, the second light source 320 is turned off, or when the first light source 310 is turned off, the second light source 320 is turned on.

Here, the control part 500 can receive a first light receiving signal of the light receiving part 400 when the first light source 310 is turned on, and the second light source 320 is turned off, receive a second light receiving signal of the light receiving part 400 when the first light source 310 is turned off, and the second light source 320 is turned on, and measure the turbidity of the fluid 110 on the basis of the first light receiving signal and the second light receiving signal.

For example, the first light receiving signal of the light receiving part 400 can be a light receiving signal generated on the basis of a plurality of scattered light including first scattered light 210 in which light emitted from the first light source 310 is primarily scattered by the suspended particles 120 of the fluid 110, the reflected light 230 in which the first scattered light 210 is reflected or re-reflected by the reflector 200, and second scattered light 220 in which the second scattered light is secondarily scattered by the suspended particles 120 of the fluid 110.

In addition, the second light receiving signal of the light receiving part 400 can be a light receiving signal generated on the basis of the first scattered light 210 in which the light emitted from the second light source 320 is primarily scattered by the suspended particles 120 of the fluid 110.

In addition, when measuring the turbidity of the fluid 110, if there is no change in the first light receiving signal due to a saturation state of the light receiving part 400, and the second light receiving signal is normally received, the control part 500 can classify the turbidity of the fluid 110 into a high turbidity region and measure a high turbidity value of the fluid 110 on the basis of the second light receiving signal.

Here, the control part 500 can measure the turbidity value of the fluid 110 within a range exceeding 1 NTU (Nethelometric Paultity part) and less than 2,000 NTU on the basis of the second light receiving signal when measuring the turbidity value of the fluid 110.

Here, the second light receiving signal can be a light receiving signal generated on the basis of the first scattered light 210 emitted from the second light source 320 and primarily scattered by the suspended particles of the fluid 110.

Next, when measuring the turbidity of the fluid 110, if there is no change in the second light receiving signal due to a saturation state of the light receiving part 400, and the first light receiving signal is normally received, the control part 500 can classify the turbidity of the fluid 110 into a low turbidity region and measure a low turbidity value of the fluid 110 on the basis of the first light receiving signal.

Here, the control part 500 can measure the low turbidity value of the fluid 110, which is 1 NTU (Nethelometric Paultity part) or less on the basis of the first light receiving signal when measuring the low turbidity value of the fluid 110.

Here, the first light receiving signal of the light receiving part 400 can be a light receiving signal generated on the basis of a plurality of scattered light including first scattered light 210 in which light emitted from the first light source 310 is primarily scattered by the suspended particles 120 of the fluid 110, the reflected light 230 in which the first scattered light 210 is reflected or re-reflected by the reflector 200, and second scattered light 220 in which the second scattered light is secondarily scattered by the suspended particles 120 of the fluid 110.

In addition, the control part 500 can determine a position of the light receiving part 400, at which the turbidity variation and the low turbidity distinction are maximized by an equation expressed by: T(x) = a₀ + aᵢP(x, θᵢ) (wherein, T(x) is an output value of a sensor for a sample having a turbidity value of x, a₀ is an initial value, aᵢ is a coefficient, and P(x, θᵢ) is a position of the light receiving part having a predetermined angle θ with respect to the fluid storage part having the turbidity value of x).

In addition, the control part 500 can analyze a pattern of an analog digital converter (ADC) of the reflector 200 at each angle centered on the fluid storage part 100 on the basis of the first light receiving signal of the light receiving part 400 to determine the position of the reflector 200 at which the turbidity variation and the low turbidity distinction are maximized.

As described above, in the present disclosure, to sense broadband turbidity including the low turbidity and the high turbidity regions, the light source and the reflector 200 can be fixed, and the light receiving part 400 can be disposed at a specific position at which the turbidity variation and the low turbidity distinction are maximized, or the light source and the light receiving part 400 can be fixed, and the reflector 200 can be disposed at a specific position at which the turbidity variation and the low turbidity distinction are maximized.

Thus, in the present disclosure, the optical signal of the low turbidity can be amplified using the reflector to enable the broadband sensing for measuring the water quality from the low turbidity region to the high turbidity region.

In addition, in the present disclosure, the safety of the drinking water can be secured by sensing the low turbidity of the water quality in the water-containing home appliances using water.

In addition, in the present disclosure, since the customized filter replacement is enabled according to the water quality pollution level, the unnecessary replacement costs can be reduced by setting the customized filter replacement cycle according to the pollution standard.

In addition, the present disclosure can be applied to the home appliances such as the water purifiers and the dishwashers that utilize the various water qualities with the broadband sensing function.

In addition, in the present disclosure, since the light source is implemented using the low-price LED, the low-price sensor can be provided and be expanded to the various home appliances.

In addition, the present disclosure can provide the water quality control and the customer assurance service through the real-time water quality monitoring and measurement.

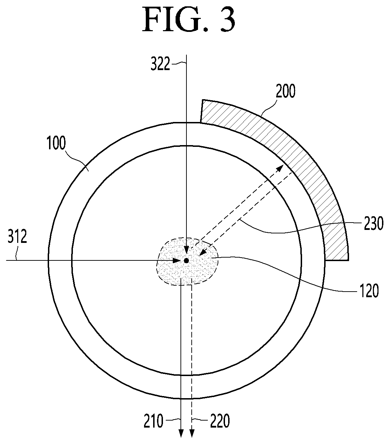

FIGS. 3 to 5 are views for explaining the fluid storage part of the device for measuring the turbidity according to an embodiment of the present disclosure.

As illustrated in FIGS. 3 to 5, the fluid storage part 100 can have a cylindrical shape in which the fluid is stored.

Here, the fluid storage part 100 can have a pipe shape having a through-hole defined therein so that the fluid can flow, but this is only an example and is not limited thereto.

As illustrated in FIG. 3, an entire surface of the fluid storage part 100 can be provided as a light-transmitting member.

The reason is to allow the first light 312 emitted from the first light source disposed at the outside and the second light 322 emitted from the second light source disposed at the outside to be incident into the internal fluid of the fluid storage part 100 so as to receive the internal scattered light into the external light receiving part.

In addition, the reflector 200 can be disposed between a first surface facing the first light source and a second surface facing the light receiving part on the fluid storage part 100.

Here, the reflector 200 can amplify the scattered light by reflecting or re-reflecting the scattered light scattered by the suspended particles 120 inside the fluid storage part 100 toward the suspended particles 120 inside the fluid storage part 100.

For example, in the present disclosure, when the first light 312 emitted from the first light source disposed at the outside is incident into the internal fluid of the fluid storage part 100, the plurality of scattered light including the first scattered light 210, in which the first light 312 emitted from the first light source is primarily scattered by the suspended particles 120 of the fluid, and the second scattered light 220 in which the reflected light 230, in which the first scattered light 210 is reflected or re-reflected by the reflector 200, is secondarily scattered by suspended particles 120 of the fluid 110 can be emitted to the external light receiving part.

In addition, in the present disclosure, when the second light 322 emitted from the second light source disposed at the outside is incident into the internal fluid of the fluid storage part 100, the first scattered light 210, in which the second light 322 emitted from the second light source is primarily scattered by the suspended particles 120 of the fluid, can be emitted to the external light receiving part.

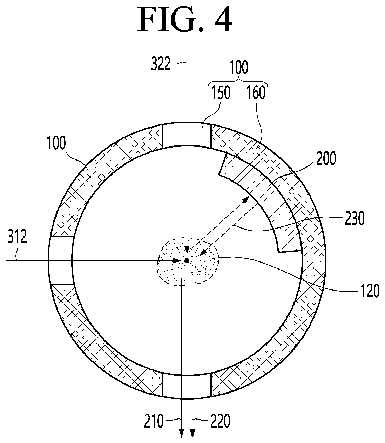

As another embodiment, as illustrated in FIG. 4, only some surfaces of the fluid storage part 100 can be provided as a light-transmitting member.

In the fluid storage part 100, the light-transmitting member 150 can be disposed only on the incident surface, through which the first light 312 and the second light 322 are incident inward from the outside, and the emission surface, through which the first and second scattered light 210 and 220 are emitted to the outside, and an opaque member 160 can be disposed on the remaining area.

Here, the reflector 200 can be attached to an inner surface of the fluid storage part 100.

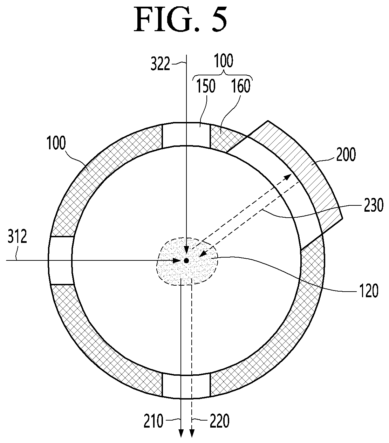

As another embodiment, as illustrated in FIG. 5, the light-transmitting member 150 can be disposed only on the incident surface, through which the first light 312 and the second light 322 are incident inward from the outside, the emission surface, through which the first and second scattered light 210 and 220 are emitted to the outside, and the attachment surface to which the reflector 200 is attached, and the opaque member 160 can be disposed on the remaining area.

Here, the reflector 200 can be attached to an outer surface of the fluid storage part 100.

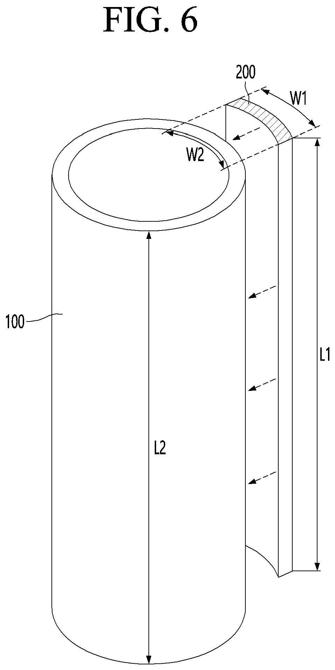

FIG. 6 is a view for explaining a reflector of the device for measuring the turbidity according to an embodiment of the present disclosure.

As illustrated in FIG. 6, the reflector 200 can amplify the scattered light by reflecting or re-reflecting the scattered light scattered by the suspended particles inside the fluid storage part 100 toward the suspended particles inside the fluid storage part 100.

That is, the reflector 200 can maximize light reflection characteristics of low turbidity particles with low light scattering.

Here, the reflector 200 can be disposed between a first surface facing the first light source and a second surface facing the light receiving part on the fluid storage part 100.

The reason is that, when the reflector 200 is disposed on an area between the first surface facing the first light source and the second surface facing the light receiving part on the surface of the fluid storage part 100, the light receiving signal of the light receiving part, by which the turbidity variation and the low turbidity distinction are maximized, can be obtained.

Thus, in the present disclosure, water quality in a low turbidity region can be accurately measured by analyzing the light receiving signal of the light receiving part, by which the turbidity variation and the low turbidity distinction are maximized.

The reflector 200 can be at least one of a retro-reflective film or a light reflective film, but this is only an example and is not limited thereto.

For example, a length L1 of the reflector 200 can be less than or equal to a length L2 of the fluid storage part 100, and a width W1 of the reflector 200 can be less than or equal to a width W2 between the first surface of the fluid storage part 100 facing the first light source 310 and the second surface of the fluid storage part 100 facing the light receiving part.

Here, an area of the reflector 200 can be calculated by a formula expressed by: S = L × W (S is an area of the reflector, L is a length of the fluid storage part, and W is a width between the first surface and the second surface of the fluid storage part), but this is only an example and is not limited thereto.

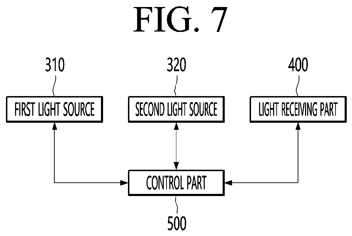

FIG. 7 is a view for explaining a method for measuring turbidity in the device for measuring the turbidity according to an embodiment of the present disclosure.

As illustrated in FIG. 7, the present disclosure can include the control part 500 that controls the first light source 310, the second light source 320, and the light receiving part 400 to measure the turbidity of the fluid.

Here, the control part 500 can alternately switch the first light source 310 and the second light source 320 so that, when the first light source 310 is turned on, the second light source 320 is turned off, or when the first light source 310 is turned off, the second light source 320 is turned on.

In addition, the control part 500 can receive the first light receiving signal of the light receiving part 400 when the first light source 310 is turned on, and the second light source 320 is turned off, receive the second light receiving signal of the light receiving part 400 when the first light source 310 is turned off, and the second light source 320 is turned on, and measure the turbidity of the fluid on the basis of the first light receiving signal and the second light receiving signal.

For example, the first light receiving signal of the light receiving part 400 can be a light receiving signal generated on the basis of the plurality of scattered light including first scattered light in which light emitted from the first light source 310 is primarily scattered by the suspended particles of the fluid, the reflected light in which the first scattered light is reflected or re-reflected by the reflector, and second scattered light in which the second scattered light is secondarily scattered by the suspended particles of the fluid.

In addition, the second light receiving signal of the light receiving part 400 can be a light receiving signal generated on the basis of the first scattered light in which the light emitted from the second light source is primarily scattered by the suspended particles of the fluid.

In addition, when measuring the turbidity of the fluid, if there is no change in the first light receiving signal due to a saturation state of the light receiving part 400, and the second light receiving signal is normally received, the control part 500 can classify the turbidity of the fluid into a high turbidity region and measure a high turbidity value of the fluid on the basis of the second light receiving signal.

Here, the control part 500 can measure the turbidity value of the fluid within a range exceeding 1 NTU (Nethelometric Paultity part) and less than 2,000 NTU on the basis of the second light receiving signal.

Next, when measuring the turbidity of the fluid, if there is no change in the second light receiving signal due to a saturation state of the light receiving part 400, and the first light receiving signal is normally received, the control part 500 can classify the turbidity of the fluid into a low turbidity region and measure a low turbidity value of the fluid on the basis of the first light receiving signal.

Here, the control part 500 can measure the low turbidity value of the fluid 110, which is 1 NTU (Nethelometric Paultity part) or less on the basis of the first light receiving signal.

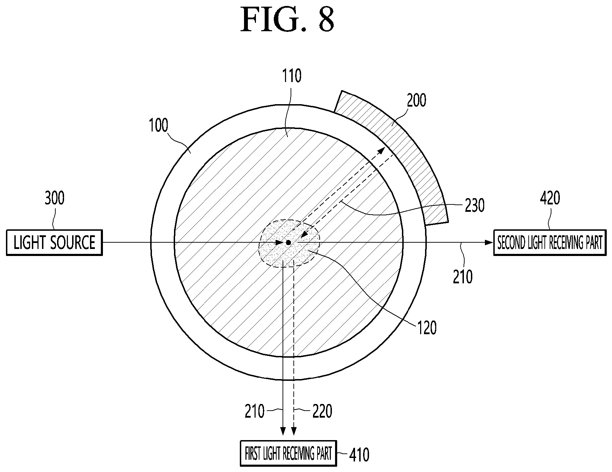

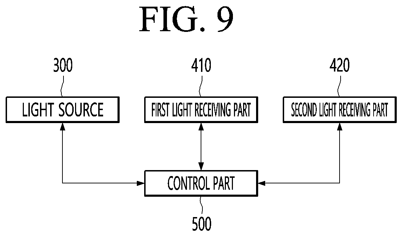

FIG. 8 is a view for explaining a device for measuring turbidity according to another embodiment of the present disclosure, and FIG. 9 is a view for explaining a method for measuring turbidity in the device for measuring the turbidity according to another embodiment of the present disclosure.

As illustrated in FIGS. 8 and 9, a device for measuring turbidity of the present disclosure can include a fluid storage part 100 including a reflector 200, a light source 300, which emits light into a fluid 110 inside the fluid storage part 100, a first light receiving part 410 and a second light receiving part 420, which receive scattered light scattered by suspended particles 120 of the fluid 110, and a control part 500 that controls the light source 300, the first light receiving part 410, and the second light receiving part 410 to measure turbidity of the fluid 110.

Here, the fluid storage part 100 can have a cylindrical shape in which the fluid is stored.

The reflector 200 can amplify the scattered light by reflecting or re-reflecting the scattered light scattered by the suspended particles inside the fluid storage part 100 toward the suspended particles inside the fluid storage part 100.

That is, the reflector 200 can maximize light reflection characteristics of low turbidity particles with low light scattering.

Here, the reflector 200 can be disposed between a first surface facing the light source 300 and a second surface facing the first light receiving part 410 on the fluid storage part 100.

The reason is that, when the reflector 200 is disposed on an area between the first surface facing the light source 300 and the second surface facing the first light receiving part 410 on the surface of the fluid storage part 100, a light receiving signal of the first light receiving part 410, by which a turbidity variation and low turbidity distinction are maximized, can be obtained.

Thus, in the present disclosure, water quality in a low turbidity region can be accurately measured by analyzing the light receiving signal of the first light receiving part 410, by which the turbidity variation and the low turbidity distinction are maximized.

Next, the light source 300 and the first light receiving part 410 can be disposed to be spaced a predetermined angle from each other around the fluid storage part 100 by using the fluid storage part 100 as a center.

For example, the light source 300 and the first light receiving part 410 can be disposed in a direction perpendicular to each other by using the fluid storage part 100 as the center.

Next, the second light receiving part 420 can be disposed to be spaced a predetermined angle from the first light receiving part 410 around the fluid storage part 100 and face the light source 300.

For example, the second light receiving part 420 can be disposed to be perpendicular to the first light receiving part 410 and face the light source 300 by using the fluid storage part 100 as a center.

That is, the light source 300 and the second light receiving part 420 can be disposed symmetrically at both sides of the fluid storage part 100 along a line passing through a center point of the fluid storage part 100.

Here, the second light receiving part 420 can be disposed on a second line having a predetermined angle with respect to the first line while passing through the center point of the fluid storage part 100 when the first light receiving part 410 is disposed on the first line passing through the center point of the fluid storage part 100.

For example, the second light receiving part 420 can be disposed on a second line perpendicular to the first line while passing through the center point of the fluid storage part 100.

Each of the first light receiving part 410 and the second light receiving part 420 of the present disclosure can be a photodiode, but this is only an example and is not limited thereto.

In addition, as illustrated in FIG. 9, the control part 500 of the present disclosure can alternatively switch the first light receiving part 410 and the second light receiving part 420 so that, when the light source 300 and the first light receiving part 410 are turned on, the second light receiving part 420 is turned off, or when the light source 300 is turned on, and the first light receiving part 410 is turned off, the second light receiving part 420 is turned on.

Here, the control part 500 can receive a first light receiving signal of the first light receiving part 410 when the first light receiving part 410 is turned on, and the second light receiving part 420 is turned off, and receive a second light receiving signal of the second light receiving part 420 when the first light receiving part 410 is turned off, and the second light receiving part 420 is turned on, and measure the turbidity of the fluid 110 on the basis of the first light receiving signal and the second light receiving signal.

For example, the first light receiving signal of the first light receiving part 410 can be a light receiving signal generated on the basis of a plurality of scattered light including first scattered light 210 in which light emitted from the light source 300 is primarily scattered by the suspended particles 120 of the fluid 110, the reflected light 230 in which the first scattered light 210 is reflected or re-reflected by the reflector 200, and second scattered light 220 in which the second scattered light is secondarily scattered by the suspended particles 120 of the fluid 110.

In addition, the second light receiving signal of the second light receiving part 420 can be a light receiving signal generated on the basis of the first scattered light 210 in which the light emitted from the light source 300 is primarily scattered by the suspended particles 120 of the fluid 110.

In addition, when measuring the turbidity of the fluid 110, if there is no change in the first light receiving signal due to a saturation state of the first light receiving part 410, and the second light receiving signal of the second light receiving part 420 is normally received, the control part 500 can classify the turbidity of the fluid 110 into a high turbidity region and measure a high turbidity value of the fluid 110 on the basis of the second light receiving signal.

Here, the control part 500 can measure the turbidity value of the fluid 110 within a range exceeding 1 NTU (Nethelometric Paultity part) and less than 2,000 NTU on the basis of the second light receiving signal of the second light receiving part 420 when measuring the turbidity value of the fluid 110.

At this time, the second light receiving signal of the second light receiving part 420 can be a light receiving signal generated on the basis of the first scattered light 210 emitted from the second light source 300 and primarily scattered by the suspended particles of the fluid 110.

Next, when measuring the turbidity of the fluid 110, if there is no change in the second light receiving signal due to a saturation state of the second light receiving part 420, and the first light receiving signal of the first light receiving part 410 is normally received, the control part 500 can classify the turbidity of the fluid 110 into a low turbidity region and measure a low turbidity value of the fluid 110 on the basis of the first light receiving signal.

Here, the control part 500 can measure the low turbidity value of the fluid 110, which is 1 NTU (Nethelometric Paultity part) or less on the basis of the first light receiving signal of the first light receiving part 410 when measuring the low turbidity value of the fluid 110.

Here, the first light receiving signal of the first light receiving part 410 can be a light receiving signal generated on the basis of a plurality of scattered light including first scattered light 210 in which light emitted from the light source 300 is primarily scattered by the suspended particles 120 of the fluid 110, the reflected light 230 in which the first scattered light 210 is reflected or re-reflected by the reflector 200, and second scattered light 220 in which the second scattered light is secondarily scattered by the suspended particles 120 of the fluid 110.

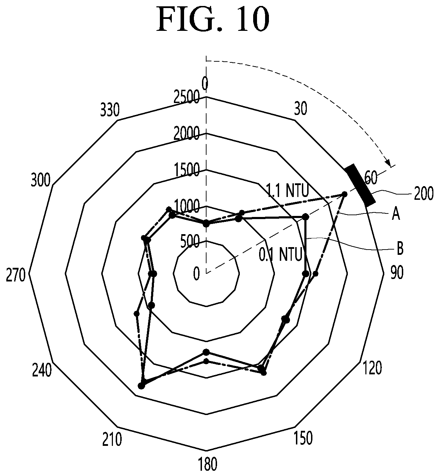

FIG. 10 is a view for explaining position setting of the reflector of the device for measuring the turbidity according to an embodiment of the present disclosure.

As illustrated in FIG. 10, in the present disclosure, a pattern of an analog digital converter (ADC) of the reflector 200 at each angle centered on the fluid storage part on the basis of the first light receiving signal of the light receiving part can be analyzed to determine a position of the reflector 200 at which the turbidity variation and the low turbidity distinction are maximized.

For example, in the present disclosure, when determining the position of the reflector 200, as illustrated in FIG. 10, a specific position at which the intensity of the light receiving signal pattern is amplified, and also, a turbidity value of 1.1 NTU of a high turbidity pattern A and a turbidity value of 0.1 NTU of a low turbidity pattern B are distinguished from each other, can be determined as the position of the reflector 200.

That is, in the present disclosure, the positions of the light source and the light receiver can be fixed, and the position of the reflector 200 can vary to fine a specific position at which the turbidity variation and the low turbidity distinction are maximized.

Thus, in the present disclosure, the optical signal of the low turbidity can be amplified using the reflector 200 to enable the broadband sensing for measuring the water quality from the low turbidity region to the high turbidity region.

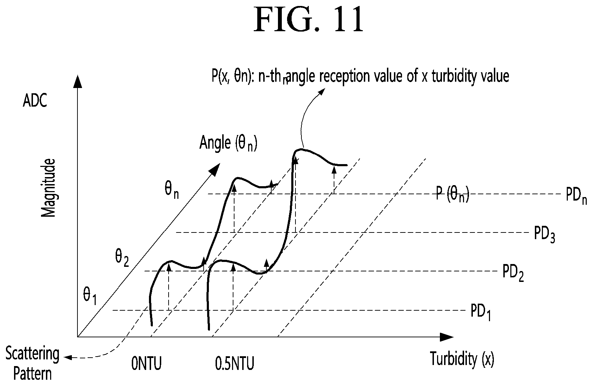

FIG. 11 is a view for explaining position setting of a light receiving part of the device for measuring the turbidity according to an embodiment of the present disclosure.

As illustrated in FIG. 11, in the present disclosure, a position of the light receiving part 400, at which the turbidity variation and the low turbidity distinction are maximized by an equation expressed by: T(x) = a₀ + aᵢP(x, θᵢ) (wherein, T(x) is an output value of a sensor for a sample having a turbidity value of x, a₀ is an initial value, aᵢ is a coefficient, and P(x, θᵢ) is a position of the light receiving part having a predetermined angle θ with respect to the fluid storage part having the turbidity value of x), can be determined.

For example, in the present disclosure, when determining the position of the light receiving part that is a photodiode, as illustrated in FIG. 11, a specific position at which the signal intensities of the high-turbidity light receiving signal pattern and the low-turbidity light receiving signal pattern are amplified, and the low turbidity is distinguished, can be determined as the position of the light receiving part on the basis of the light receiving signal pattern for each turbidity.

That is, in the present disclosure, the positions of the light source and the reflector can be fixed, and the position of the light receiving part can vary to find a specific position at which the turbidity variation and the low turbidity distinction are maximized.

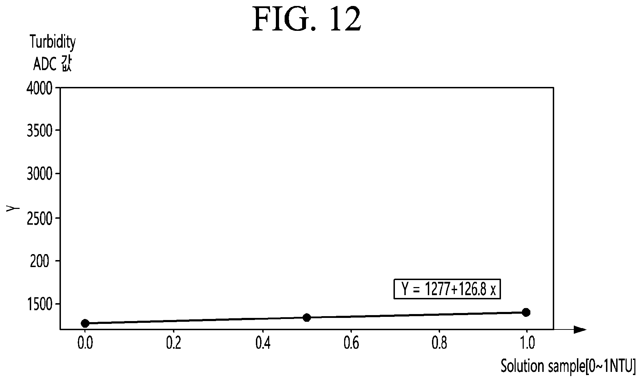

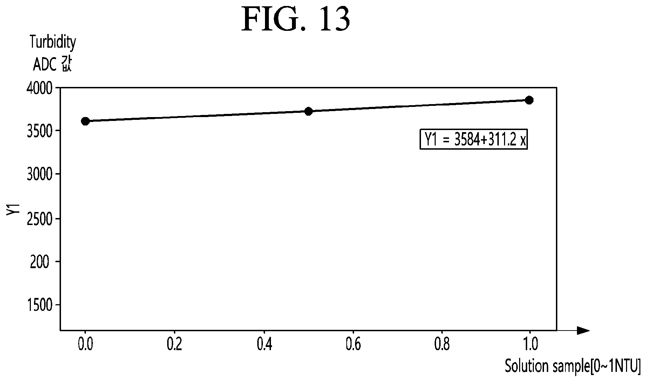

FIGS. 12 and 13 are views for explaining corresponding optical signal amplification before and after the reflector is applied to the device for measuring the turbidity according to an embodiment of the present disclosure.

FIG. 12 is a view for explaining corresponding optical signal amplification before the reflector of the present disclosure is applied, and FIG. 13 is a view for explaining corresponding optical signal amplification after the reflector of the present disclosure is applied.

As illustrated in FIG. 12, if the reflector is not applied, when the ACDC value of the light receiving signal is measured according to turbidity of a solution sample, since a turbidity inclination showing the light amplification appears to be approximately 126.8, it is difficult to distinguish a low turbidity signal value and a high turbidity signal value from each other, and thus low turbidity measurement is impossible.

In contrast, as illustrated in FIG. 13, if the reflector is applied, when the ACDC value of the light receiving signal is measured according to the turbidity of the solution sample, since the turbidity inclination showing the light amplification is improved to approximately 311.2, the low turbidity signal value and the high turbidity signal value can be distinguished from each other, and thus the low turbidity measurement is possible.

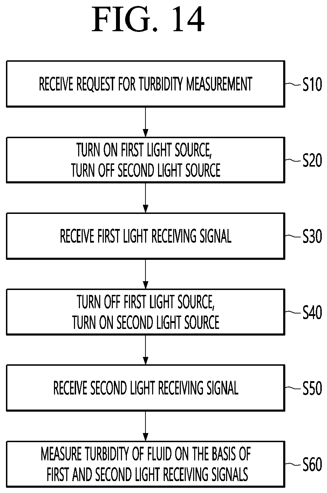

FIG. 14 is a view for explaining a method for measuring turbidity in the device for measuring the turbidity according to an embodiment of the present disclosure.

As illustrated in FIG. 14, in the present disclosure, a user input requesting turbidity measurement can be received (S10).

In addition, in the present disclosure, when the user input is received, a first light source can be turned on, and a second light source can be turned off (S20).

Next, in the present disclosure, a first light receiving signal can be received from a light receiving part (S30).

Here, the first light receiving signal of the light receiving part can be a light receiving signal generated on the basis of a plurality of scattered light including first scattered light in which light emitted from the first light source is primarily scattered by suspended particles of a fluid, reflected light in which the first scattered light is reflected or re-reflected by the reflector, and second scattered light in which second scattered light is secondarily scattered by the suspended particles of the fluid.

Next, in the present disclosure, the first light source can be turned off, and the second light source can be turned on (S40).

In addition, in the present disclosure, a second light receiving signal can be received from the light receiving part (S50).

Here, the second light receiving signal can be a light receiving signal generated on the basis of the first scattered light in which the light emitted from the second light source is primarily scattered by the suspended particles of the fluid.

Next, in the present disclosure, turbidity of the fluid can be measured on the basis of the first light receiving signal and the second light receiving signal (S60).

Here, in the present disclosure, when the turbidity of the fluid is measured, the control part can be configured to classify the turbidity of the fluid into a high turbidity region to measure a high turbidity value of the fluid on the basis of the second light receiving signal when there is no change in the first light receiving signal due to a saturation state of the light receiving part, and the second light receiving signal is normally received.

In addition, in the present disclosure, when the turbidity of the fluid is measured, the control part can be configured to classify the turbidity of the fluid into a low turbidity region to measure a low turbidity value of the fluid on the basis of the first light receiving signal when there is no change in the second light receiving signal due to the saturation state of the light receiving part, and the second light receiving signal is normally received.

As described above, the device for measuring the turbidity according to the present disclosure can amplify the optical signal of the low turbidity using the reflector to enable the broadband sensing for measuring the water quality from the low turbidity region to the high turbidity region.

In addition, in the present disclosure, the safety of the drinking water can be secured by sensing the low turbidity of the water quality in the water-containing home appliances using water.

In addition, in the present disclosure, since the customized filter replacement is enabled according to the water quality pollution level, the unnecessary replacement costs can be reduced by setting the customized filter replacement cycle according to the pollution standard.

In addition, the present disclosure can be applied to the home appliances such as the water purifiers and the dishwashers that utilize the various water qualities with the broadband sensing function.

In addition, in the present disclosure, since the light source is implemented using the low-price LED, the low-price sensor can be provided and be expanded to the various home appliances.

In addition, the present disclosure can provide the water quality control and the customer assurance service through the real-time water quality monitoring and measurement.

### INDUSTRIAL APPLICABILITY

According to the device for measuring the turbidity according to the present disclosure, the low turbidity optical signal can be by amplified using the reflector to enable the broadband sensing for measuring the water quality from the low turbidity region to the high turbidity region, and therefore, the industrial applicability is remarkable.

## Claims

1. A device for measuring turbidity, comprising:
a fluid storage part comprising a reflector;
a first light source configured to emit light to a fluid within the fluid storage part;
a first light receiving part configured to receive scattered light scattered by suspended particles in the fluid; and
a control part configured to measure turbidity of the fluid by controlling the first light source and the first light receiving part,
wherein the first light source and the first light receiving part are disposed to be spaced a predetermined angle from each other around the fluid storage part in the vicinity of the fluid storage part, and
the reflector is disposed between a first surface facing the first light source and a second surface facing the first light receiving part on a surface of the fluid storage part.

2. The device according to claim 1, wherein the reflector is configured to reflect or re-reflect the scattered light onto the suspended particles of the fluid, thereby amplifying the scattered light.

3. The device according to claim 1, further comprising a second light source configured to emit light to the fluid within the fluid storage part,
wherein the second light source is spaced a predetermined angle from the first light source around the fluid storage part and disposed to face the first light receiving part.

4. The device according to claim 3, wherein each of the first light source and the second light source comprises a light emitting diode.

5. The device according to claim 3, wherein the control part is configured to:
turn off the second light source when the first light source is turned on; or
alternately switch the first light source and the second light source so that the second light source is turned on when the first light source is turned off.

6. The device according to claim 5, wherein the control part is configured to:
receive a first light receiving signal of the first light receiving part when the first light source is turned on, and the second light source is turned off;
receive a second light receiving signal of the first light receiving part when the first light source is turned off, and the second light source is turned on; and
measure the turbidity of the fluid on the basis of the first light receiving signal and the second light receiving signal.

7. The device according to claim 6, wherein the first light receiving signal of the first light receiving part is a light receiving signal on the basis of a plurality of scattered light comprising first scattered light, in which light emitted from the first light source is primarily scattered by the suspended particles of the fluid, and second scattered light, in which the first scattered light is reflected or re-reflected by the reflector and is secondarily scattered by the suspended particles of the fluid, and
the second light receiving signal of the first light receiving part is a light receiving signal generated on the basis of the first scattered light, in which light emitted from the second light source is primarily scattered by the suspended particles of the fluid.

8. The device according to claim 6, wherein, when the turbidity of the fluid is measured, the control part is configured to classify the turbidity of the fluid into a high turbidity region to measure a high turbidity value of the fluid on the basis of the second light receiving signal when there is no change in the first light receiving signal due to a saturation state of the first light receiving part, and the second light receiving signal is normally received.

9. The device according to claim 6, wherein, when the turbidity of the fluid is measured, the control part is configured to classify the turbidity of the fluid into a low turbidity region to measure a low turbidity value of the fluid on the basis of the first light receiving signal when there is no change in the second light receiving signal due to a saturation state of the first light receiving part, and the second light receiving signal is normally received.

10. The device according to claim 1, further comprising a second light receiving part configured to receive the scattered light scattered by the suspended particles of the fluid,
wherein the second light receiving part is spaced a predetermined angle from the first light receiving part around the fluid storage part and disposed to face the first light receiving part.

11. The device according to claim 10, wherein each of the first light receiving part and the second light receiving part comprises a photodiode.

12. The device according to claim 10, wherein the control part is configured to:
turn off the second light receiving part when the first light source and the first light receiving part are turned on; or
alternatively switch the first light receiving part and the second light receiving part so that the second light receiving part is turned on when the first light source is turned on, and the first light receiving part is turned off.

13. The device according to claim 12, wherein, when the turbidity of the fluid is measured, the control part is configured to classify the turbidity of the fluid into a high turbidity region to measure a high turbidity value of the fluid on the basis of the second light receiving signal when there is no change in the first light receiving signal due to a saturation state of the first light receiving part, and the second light receiving signal of the second light receiving part is normally received.

14. The device according to claim 12, wherein, when the turbidity of the fluid is measured, the control part is configured to classify the turbidity of the fluid into a low turbidity region to measure a low turbidity value of the fluid on the basis of the first light receiving signal when there is no change in the second light receiving signal due to a saturation state of the second light receiving part, and the second light receiving signal of the first light receiving part is normally received.

15. A method for measuring turbidity using a device for measuring turbidity, which comprises a first light source, a second light source, and a light receiving part, which are disposed at predetermined angles around a fluid storage part comprising a reflector, the method comprising:
receiving an user input requesting a turbidity measurement;
turning on the first light source and turning off the second light source when the user input is received;
receiving a first light receiving signal from the light receiving part:
turning off the first light source and turning on the second light source;
receiving a second light receiving signal from the light receiving part: and
measuring the turbidity of the fluid on the basis of the first light receiving signal and the second light receiving signal.
